(19) Europäisches Patentamt
European Patent Office
Office européen des brevets

(11) Publication number : **0 628 545 A1**

(12) # EUROPEAN PATENT APPLICATION

(21) Application number : **94304299.4**

(51) Int. Cl.[5] : **C07C 405/00,** // A61K31/557

(22) Date of filing : **14.06.94**

(30) Priority : **14.06.93 JP 167531/93**

(43) Date of publication of application :
**14.12.94 Bulletin 94/50**

(84) Designated Contracting States :
**AT BE CH DE DK ES FR GB GR IE IT LI LU MC NL PT SE**

(71) Applicant : **ONO PHARMACEUTICAL CO., LTD.**
**1-5, Doshomachi 2-chome**
**Chuo-ku Osaka 541 (JP)**

(72) Inventor : **Miyazaki, Tohru c/o Ono Pharmaceutical Co. Ltd.**
**Minase Research Institute,**
**1-1, Sakurai 3-chome,**
**Shimamoto-cho, Mishima-gun, Osaka 618 (JP)**
Inventor : **Kawakura, Masanori c/o Ono Pharmaceutical Co. Ltd.**
**Minase Research Institute,**
**1-1, Sakurai 3-chome,**
**Shimamoto-cho, Mishima-gun, Osaka 618 (JP)**
Inventor : **Shirasawa, Eiichi**
**1-1, Ohe Kitafukunishi-cho 4-chome,**
**Nishikyo-ku, Kyoto 610-11 (JP)**

(74) Representative : **Collier, Jeremy Austin Grey**
**J.A.KEMP & CO.**
**14, South Square**
**Gray's Inn**
**London WC1R 5LX (GB)**

(54) **13,14-Dihydro-PGF2BETA and its isopropyl ester.**

(57) 13,14-dihydro-prostaglandin F2β and its isopropyl ester of the formula :

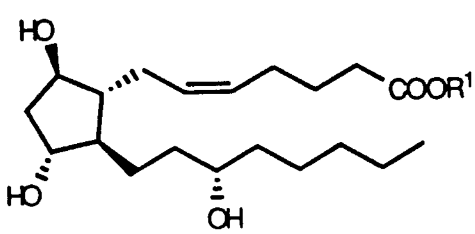

(I)

reduce intraocular pressure at low concentration and have low stimulus and are therefore useful for preventing and/or treating glaucoma.

EP 0 628 545 A1

The present invention relates to prostaglandins and their esters.

More particularly, the present invention provides 13,14-dihydro-PGF2β and its isopropyl ester of formula:

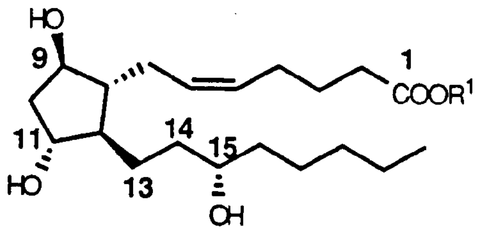

(I)

wherein $R^1$ is hydrogen or isopropyl, and their cyclodextrin clathrates and, when $R^1$ is hydrogen, its pharmaceutically acceptable salts. These compounds are useful for prevention and/or treatment of glaucoma.

Glaucoma is a disease caused by elevation of intraocular pressure. Enhancement of the illness causes contraction of the visual field and ultimately blindness. Intraocular pressure is changed by the balance of the secretion of aqueous humor from the ciliary body epithelium and its excretion from Schlemm's cannal. Imbalance causes elevation of intraocular pressure.

Treatment for glaucoma is currently carried out by two different methods, i.e. by surgical operation or by administration of reducing agent of intraocular pressure. As reducing agents of intraocular pressure, miotic agents (pilocarpine, carbacol etc.), carbonic anhydrase inhibitors and β-blockers are known.

It is known that certain derivatives of prostaglandins (PG) possess a reducing activity of intraocular pressure without heavy side effects.

For example, it has been disclosed that PGF2α alkyl esters of the formula:

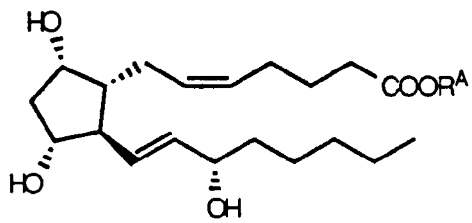

(A)

wherein $R^A$ is C1-5 alkyl, possess reducing activity of intraocular pressure (see Japanese Patent Kokai No. 59-1418 or European Patent Publication No. 93,380). In this specification, PGF2α methyl ester, PGF2α ethyl ester, PGF2α isopropyl ester, PGF2α, PGF2β, 15-keto-PGF2α, 16,16-dimethyl-PGF2α and PGF1α are specifically disclosed as effective PGF type compounds.

It has also been disclosed that 11-epi-PGF2α alkyl and alkyl-aryl esters of formula:

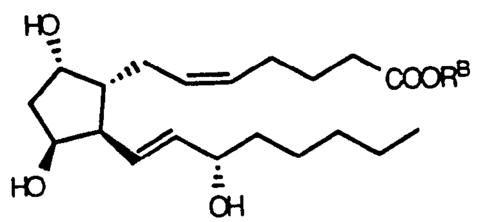

(B)

wherein $R^B$ is alkyl or alkyl-aryl, possess reducing activity of intraocular pressure (see Japanese Patent Kokai No. 2-501483 or European Patent Publication No. 344,235). In this specification, 11-epi-PGF2α isopropyl ester is specifically disclosed as an effective compound.

Further, it has been disclosed that 15-acyl-PG derivatives of formula:

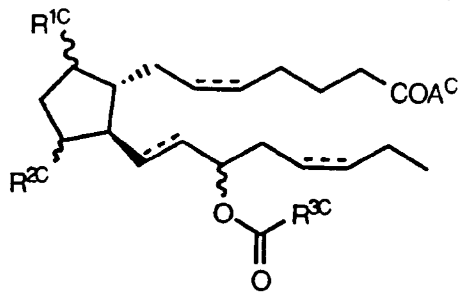

(C)

wherein dotted lines are a bond or cis- or trans-double bond, $A^C$ is OH or a pharmaceutically acceptable salt or $OR^{5C}$, $R^{1C}$ and $R^{2C}$ are the following combination: -OH/-OH, =O/-OH or -OH/=O, $R^{3C}$ is non-cyclic saturated or unsaturated hydrocarbon of C1-20 or -(CH2)n-$R^{4C}$ (wherein n is 0-10, $R^{4C}$ is C3-7 aliphatic ring or aromatic or hetero-aromatic ring and $R^{5C}$ is C1-10 aliphatic group), possess reducing activity of intraocular pressure (see Japanese Patent Kokai No. 3-17017 or European Patent Publication No. 399,839). In this specification, 15-acetyl-PGF2α, 15-isobutyryl-PGF2α, 15-valeryl-PGF2α, 15-isovaleryl-PGF2α, 15-pivaloyl-PGF2α, PGF2α are specifically disclosed as effective compounds.

On the other hand, it has been disclosed that 13,14-dihydro-PGF2α derivatives of formula:

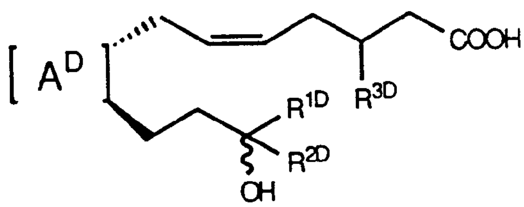

(D)

wherein $A^D$ is formula:

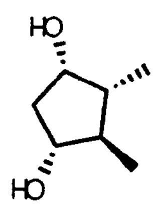

wherein $R^{1D}$ is formula:

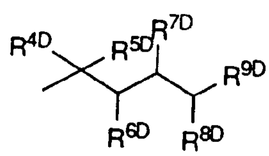

wherein $R^{4D}$ and $R^{5D}$ is the same or different, hydrogen or C1-4 alkyl, $R^{6D}$ is hydrogen or C1-3 alkyl, $R^{7D}$ is hydrogen, methyl or ethyl, $R^{8D}$ is hydrogen or methyl, $R^{9D}$ is hydrogen or methyl, with the proviso that three of $R^{4D}$, $R^{5D}$, $R^{6D}$, $R^{7D}$ and $R^{8D}$ are not alkyl groups at the same time, $R^{1D}$ is methyl, ethyl, propyl or butyl having cyclohexyl or cyclopentyl, $R^{2D}$ is hydrogen or C1-4 alkyl, $R^{3D}$ is hydrogen, with the proviso that, when $R^{2D}$ is hydrogen, $R^{1D}$ is not n-pentyl, and esters thereof, cyclodextrin clathrates thereof and non-toxic acid salts thereof, possess hypotensive activity, inhibitory activity on gastric acid secretion and gastric ulceration, bronchodilating activity, leuteolytic activity, stimulatory activity on uterine contraction and on intestinal contraction (see British Patent No. 1,450,691).

And it has been disclosed that 13,14-dihydro-PGF2α of formula:

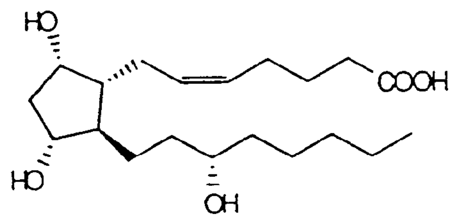

(E)

is a small quantity metabolite of PGF2α in serum (see E. Granstrom, Eur. J. Biochem., 27, 462 (1972)). In this literature, 13,14-dihydro-PGF2α methyl ester for structural assignment of 13,14-dihydro-PGF2α is also disclosed.

PGF2β ethyl ester of formula:

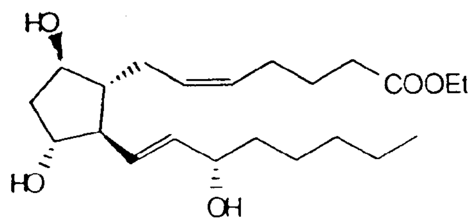

(F)

is disclosed specifically in the specification of French Patent No. 2,290,425, but only as a starting material.

The present inventors have measured the reducing activity of intraocular pressure of various known PGF type compounds and found that all of them possess weak activity or have side-effects (hyperemia of iris or conjunctiva, initial elevating activity of intraocular pressure, general reaction etc.). To avoid side effects, reduction of dose or concentration of the administration reduces the main activity. So it was difficult to separate main activity and side effects.

The present inventors have synthesized many kinds of PGF type compounds and investigated their pharmacological activities and physical properties, and have found that 13,14-dihydro-PGF2β isopropyl ester possesses strong reducing activity on intraocular pressure, has low side effects and good physical properties.

The structural characters of the compounds of the present invention are as follows:

1) hydroxy group on 9-position are bonded with β-configuration on the ring;

2) double bond between 13 and 14-positions is saturated; and

3) carboxy moiety of natural PGF2α is modified into isopropyl ester.

The present invention includes not only 13,14-dihydro-PGF2β isopropyl ester but also 13,14-dihydro-PGF2β which is the free acid of the ester. It has been confirmed that this free acid is a metabolite of the 13,14-dihydro-PGF2β isopropyl ester, and it is thought that the free acid is the active compound of reducing activity of intraocular pressure.

Prostaglandins wherein the double bond between 13 and 14-position is saturated and hydroxy group on 9-position bonded with β configuration are not known in the literature.

The compounds of the present invention are not included in the formula (A) or (B), as the compounds have structures wherein 9-hydroxy group is attached with α-configuration and the 13 and 14 positions are double bonded.

In the formula (C), compounds wherein $R^{1C}$ are attached to β-configuration on the ring and C13-14 is single bonded are included. But, in the specification 13,14-dihydro-PGF2β type compounds are not disclosed specifically. The specification does not disclose 13,14-dihydro-PGF2β, but only discloses specifically 15-acetyl-PGF2α, 15-isobutyl-PGF2α, 15-valeryl-PGF2α, 15-isovaleryl-PGF2α, 15-pivaloyl-PGF2α and PGF2α as PGF type compounds.

And further, the compounds of the formula (C) are different to the compounds of the present invention at the point that 15-position hydroxy group must be esterified. It cannot be predicted that the compound of the present invention in which 15 position is natural hydroxy, is superior to the compounds of the formula (C) which is characterized by the modification of the hydroxy of 15 position, at the point of reducing activity of intraocular pressure, side effects and physical properties.

And, the formula (C) includes α- (natural) or β-(non-natural) configuration on 9-position of hydroxy group, but, this application discloses only α-configuration compounds specifically, and no disclosure of compounds of β-configuration specifically. The present invention is related that compounds of 9β-configuration possess good main activity and lower side effects. The above application does not suggest the present invention.

The compounds of the formula (D) are different to the compound of the present invention at the point of

the α-configuration of 9 position hydroxy group.

And only free acids are disclosed in the application i.e. 20-nor-13,14-dihydro-PGF2α and 16(R)-methyl-13,14-dihydro-PGF2α as 13,14-dihydro-PGF2α derivatives. In the application, esters are not synthesized.

It is also disclosed in the application that 13,14-dihydro-PGF2α derivatives possess hypotensive activity, inhibitory activity on gastric acid secretion and gastric ulceration, bronchodilating activity, leuteolytic activity, stimulatory activity on uterine contraction and on intestinal contraction. But, these descriptions do not suggest the reducing activity of intraocular pressure of the present invention.

The compounds of the present invention are different to the compounds of the formula (E) at the point that 9-position hydroxy group is bonded with α-configuration to the ring. In the specification, 13,14-dihydro-PGF2α is described only as a metabolite of PGF2α in serum, and 13,14-dihydro-PGF2α methyl ester is used as a structural confirmation for 13,14-dihydro-PGF2α. The descriptions in the specification do not suggest the reducing activity of intraocular pressure of the compounds of the present invention.

And, PGF2β ethyl ester of formula (F) is disclosed specifically in the specification of French Patent No. 2,290,425. But, this compound is only disclosed as a starting material. So, the disclosure does not suggest the present invention.

In the above points, the compound of the present invention, i.e. 13,14-dihydro-PGF2β isopropyl ester, is novel. And it is impossible to predict that the compound of the present invention, i.e. 13,14-dihydro-PGF2β and its isopropyl ester possess strong activity of reducing intraocular pressure, low side effects and have good physical properties from the above related arts.

The compounds of the present invention are 13,14-dihydro-PGF2β and its isopropyl ester of the formula (I):

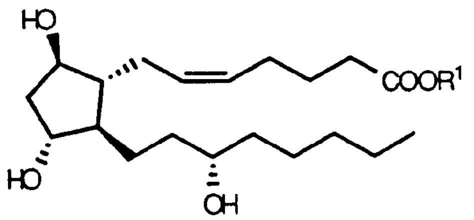

(I)

wherein $R^1$ is hydrogen or isopropyl, and pharmaceutically acceptable salts thereof, and their cyclodextrin clathrates.

The compounds of the formula (I) wherein $R^1$ is hydrogen may be converted into the corresponding salts by methods known <u>per se</u>. Non-toxic and water-soluble salts are preferable. Suitable salts are, for example, salts of alkali metals (potassium, sodium etc.), salts of alkaline earth metals (calcium, magnesium etc.), zinc, ammonium salts, salts of pharmaceutically-acceptable organic amine (tetramethylammonium, triethylamine, methylamine, dimethylamine, cyclopentylamine, benzylamine, phenethylamine, piperidine, monoethanolamine, diethanolamine, tris(hydroxymethyl)aminomethane, lysine, arginine, caffeine, N-methyl-D-glucamine etc.).

Cyclodextrin clathrates of 13,14-dihydro-PGF2β and its isopropyl ester of the formula (I) may be prepared by the method described in British Patent Nos. 1,351,238 or 1,419,221, using α-, β- or γ- cyclodextrins or mixture thereof. Converting into their cyclodextrin clathrates serves to increase the stability and solubility in water of the compounds, and therefore facilitates their administration as pharmaceuticals.

Among the compounds of the formula (I), a compound wherein $R^1$ is isopropyl, of the formula (IB):

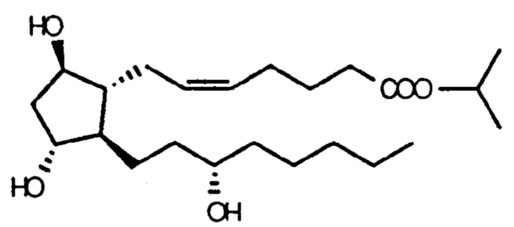

(IB)

may be prepared by hydrolysing a compound of formula (II):

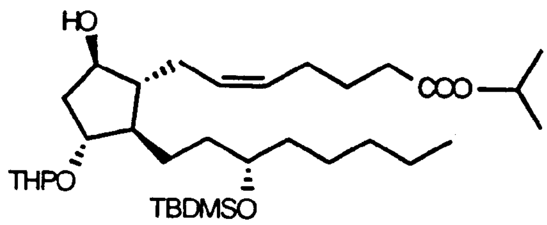

(II)

wherein THP is tetrahydropyran-2-yloxy, and TBDMS is t-butyldimethylsilyl, under acidic conditions.

Hydrolysis under acidic conditions is known. It may be carried out, for example, in water-miscible organic solvent (tetrahydrofuran, dioxan, methanol, ethanol etc.) using acid (acetic acid, trichloroacetic acid, hydrochloric acid, oxalic acid etc.), at a temperature of 0-90 °C.

The compound of the formula (II) may be prepared by the following reaction scheme (1).

Reaction Scheme (1)

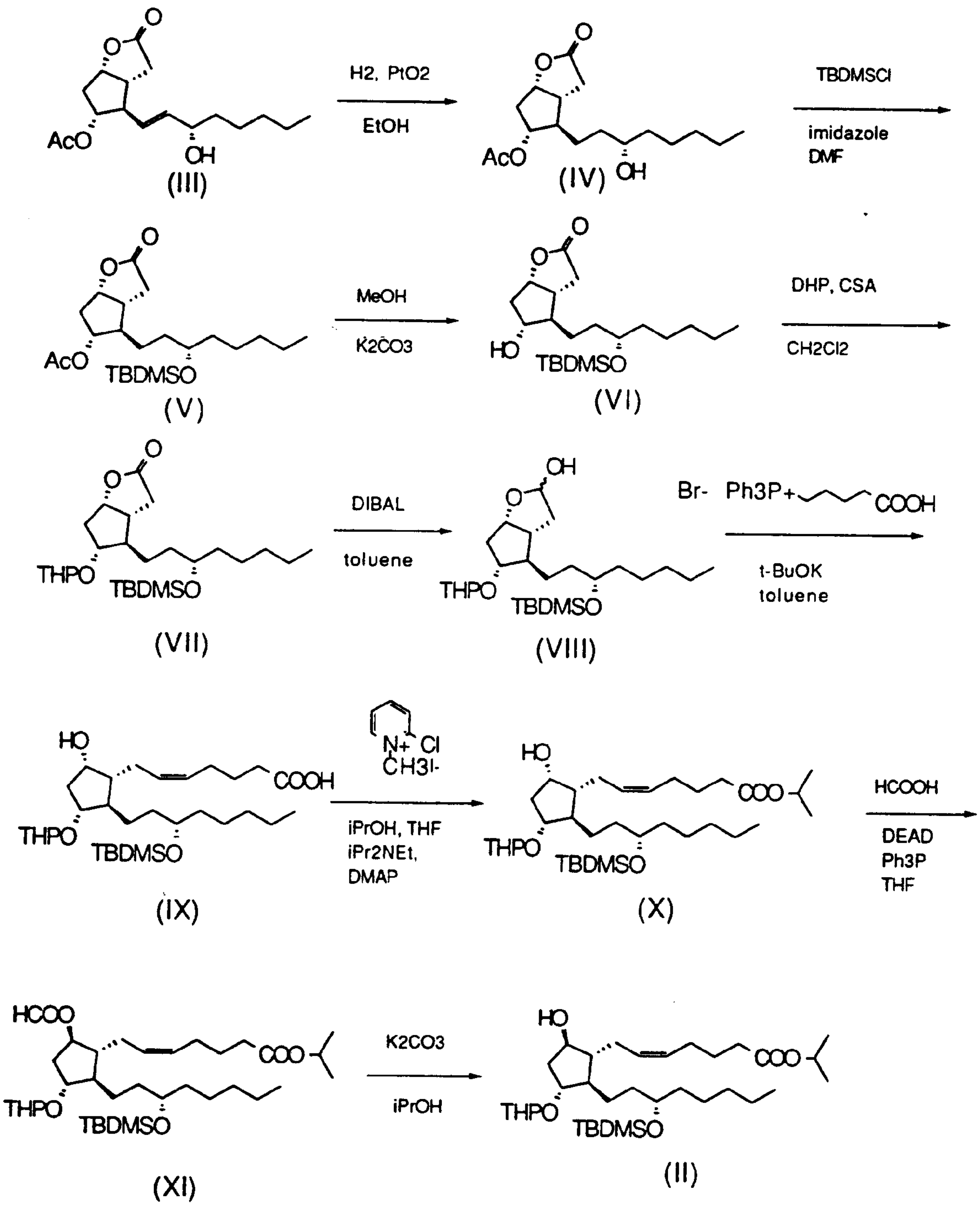


(III) (IV) (V) (VI) (VII) (VIII) (IX) (X) (XI) (II)

In the reaction scheme (1), TBDMS is t-butyldimethylsilyl;
THP is tetrahydropyran-2-yl;
DMF is dimethylformamide;
DHP is 3,4-dihydro-2H-pyran;
CSA is camphorsulfonic acid;
DIBAL is diisobutyl aluminum hydride;

THF is tetrahydrofuran;
DMAP is 4-methylaminopyridine;
DEAD is diethyl azocarboxylate.

The starting material of the formula (III) i.e. 2-oxa-3-oxo-6-syn-(3'α-hydroxy-1'-trans-octenyl)-7-anti-acetoxy-cis-bicyclo-[3.3.0]octane is a known compound (see J. Am. Chem. Soc., 91, 5675 (1969)).

Among the compounds of the present invention of the formula (I), a compound wherein $R^1$ is hydrogen, of formula (IA):

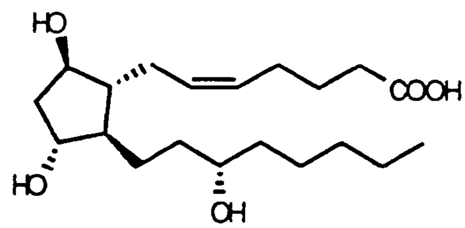

(IA)

may be prepared by hydrolysing a compound of the formula (XI), under acidic conditions and further hydrolysing the resulting compound under alkaline conditions.

Hydrolysis under acidic conditions may be carried out by the method described hereinbefore.

Hydrolysis under alkaline conditions is a known reaction. It may be carried out, for example, in a water-miscible solvent (methanol, ethanol, ether etc.), using an aqueous alkaline solution (potassium hydroxide, sodium hydroxide, lithium hydroxide etc.), at room temperature to 80°C.

The compound of the formula (IB) may be prepared by hydrolysing the compounds of the formula (IA) in alkaline conditions. This hydrolysis may be carried out by the method described hereinbefore.

## Pharmacological Activity

The compound of the present invention of the formula (I) possesses a reducing activity of intraocular pressure as described before.

## Experiment 1

For example, in a standard laboratory test the following data shown in Table 1 and 2 and in the accompanying Figure 1 were obtained. Average of the value of three samples are shown as each data. In the Figure reduction in intraocular pressure is plotted against time after administration for the compound of the invention and three comparison compounds.

## Experimental Method

Male Japanese White rabbits weighing about 3.0 kg were lightly held and the intraocular pressure was measured by Applanation Pneumatonograph after corneal anesthesia with instillation of 0.4% oxybuprocaine hydrochloride solution.

A 0.1% solution of a test compound was dissolved in vehicle which contains 2.5% conc. glycerol and 2.0% Polysorbate 80 in sterile purified water. The test solution at volume of 50 µl was instilled into left eye of each rabbit and the right eye remained untreated. As a control, the vehicle was instilled into left eye in a control group. Δ IOP (mmHg), which is an index of intraocular pressure-reducing effect, were calculated using below formula.

$$IOP = (IOP_t - IOP_0) - (IOP_{vt} - IOP_{v0})$$

$IOP_t$ : IOP at t hr after instillation of a test solution
$IOP_{vt}$ : IOP at t hr after instillation of vehicle
$IOP_0$ : IOP just before instillation of a test solution
$IOP_{v0}$ : IOP just before instillation of vehicle

Table 1 : Reducing Effects of Intraocular Pressure

| Time after administration (hr) | 0 | 1 | 2 | 4 | 6 |
|---|---|---|---|---|---|
| Example 1 | 0 | -1.8 | -5.5 | -5.5 | -5.3 |
| Compared compound 1 | 0 | 3.5 | 1.5 | 1.5 | 0.2 |
| Compared compound 2 | 0 | 13.7 | 3.7 | -1 | -5.8 |
| Compared compound 3 | 0 | 3.3 | -1.3 | -0.7 | -4.3 |

(mm Hg)

Table 2 :

| Reducing Effects of Intraocular Pressure | |
|---|---|
| Compounds | Change in I.O.P. (mmHg x hr) |
| Example 1 | -26.35 |
| Compared compound 1 | 8.95 |
| Compared compound 2 | 11.36 |
| Compared compound 3 | -4.35 |

Result

As shown in Table 1 and Fig. 1, a 0.1% solution of the compound of the present invention reduced intraocular pressure immediately after the instillation and the maximum reduction of IOP was 5.5 mmHg. And when the compound of the present invention was instilled, bulbar and palpebral conjunctival hyperemia were not observed.

This results showed that the compound of the present invention had a strong and long lasting intraocular pressure-reducing effect. The compound of the present invention is excellent and effective enough for pharmaceutical use, free from side-effects, and with satisfactory physical properties.

Compared compounds 1, 2 and 3 are the following:

Compared compound 1 -- 11-epi-PGF2α isopropyl ester which is described in experiment 1 of Japanese Patent Kokai No. 2-501483 i.e. EP-344,235

Compared compound 2 -- PGF2α isopropyl ester which is described in the 3rd Table of Japanese Patent Kokai No. 59-1418 i.e. EP-93,380

Compared compound 3 -- 13,14-dihydro-PGF2α methyl ester which is described in E. Granstrom, Eur. J. Biochem., 27, 462 (1972)

Discussion

As anti-glaucoma agent, following properties are required:

1) reduction of intraocular pressure shall occur rapidly, and be continued,

2) less side effects such as initial elevation of intraocular pressure.

We compared the compound of the present invention (example 1) and compared compounds 1, 2 and 3 from the above view points.

First, comparision is done from the view point 1). The compound of the present invention (example 1) showed:

a. The effect appeared at 1hr after administration.

b. The activity reached 5.5 mmHg at 2 hrs after administration and same level activity was maintained till

6 hrs after administration.

Compared compounds 2 and 3 showed:

a. The effect appeared barely at 4 hrs after administration.

b. The activity reached to the same level of at 2 hrs after administration of the compound of the present invention at 6 hrs after administration.

Compared compound 1 showed that the effect did not yet appear 6 hrs after administration yet.

Taking into consideration the continuous time of occurrence of the reduction effect and the intensity of the effect, the superiority of the compound of the present invention to the compared compound is clear. On the graph 1, the squares i.e. reducing effect of intraocular pressure, were calculated: That is, squares under 0 mmHg put minus and squares over 0 mmHg put plus, and the total squares are calculated and the values are shown in the Table 2. From the values of the Table 2, it is clear that the compound of the present invention is superior to the compared compounds.

Next, comparision is done from the view point 2). The compound of the present invention (example 1) did not show initial elevation of the intraocular pressure. The compared compounds 1 and 3 both showed initial elevation. Compared compound 1 possessed continuous elevation till 6 hrs after administration. And compared compound 2 also possessed extremely strong initial elevation. From the view point of the initial elevation, it is clear that the compound of the present invention is superior to these compared compounds.

On the graph of the intraocular pressure in course of time, area of the square (mmHg x hr) in elevation i.e. square over 0 mmHg and area of the square (mmHg x hr) in the reduction i.e. square under 0 mmHg were calculated and from the value of the former was subtracted the one of the latter. The minus-resulted value means reduction of intraocular pressure and the plus-resulted value means elevation of intraocular pressure. The bigger the minus value the compound possesses, the better the compound is for the reducing effect of intraocular pressure.

## Experiment 2

### Method

Japanese White rabbits weighing 3.0 kg were used. Eye drop solution of Example 1 was administered three times every 5 mins in saccus conjunctivae. 1 Hr after the last administration, aqueous humor, cornea and iris/ciliary body were collected and homogenized respectively. The homogenates were measured by liquid chromatography-Mass spectrum.

### Result

The compound of the present invention (example 1) was not detected and only the compound of Example 2 was detected in the homogenates of aqueous humor, cornea or iris/ciliary body. Almost all of the compound of Example 1 is hydrolysed into the compound of Example 2 on cornea and the compound moves into internal eye. It is therefore believed that the free acid also possesses the activity of reducing intraocular pressure, like the isopropyl ester.

### Toxicity

The toxicity of the compounds of the present invention is very low and therefore, it may be confirmed that the compounds of the present invention are safe for pharmaceutical use.

### Application for Pharmaceuticals

To reduce ocular pressure is effective method for prevention and/or treatment of glaucoma in animals, especially human beings.

The compounds of the present invention possess the activity of reducing intraocular pressure without side effects such as stimulus to eyes, from the results of in vivo experiment, so it is effective for the prevention and/or treatment of glaucoma.

For the purpose above described, the compound of the present invention of the formula (I), may be used normally as a solution. The solutions may be prepared by the technique usually used.

For example, eye drops may be prepared by adding to sterilized and purified water, isotonic buffer (sodium chloride, concentrated glycerol etc.), buffer (sodium phosphate etc.), dissolving agent (Polysorbate 80 etc.), stabilizing agent (sodium edetate etc.) and/or preserving agent (benzalkonium chloride etc.) etc. as necessary.

The doses to be administered in the form of eye drops are determined depending upon symptom, age, the desired therapeutic effects etc. As normally used, the concentration of the solution is between 0.0001% and 0.5%. A preferable concentration of the solution is between 0.001% and 0.2%.

The following reference examples and examples illustrate the present invention. The solvents in the parentheses show the developing or eluting solvents and the ratios of the solvents used are by volume in chromatographic separation.

Unless otherwise specified, "IR" was measured by KBr tablet method and "NMR" was measured in chloroform-d, respectively.

Reference Example 1

Synthesis of 2-oxa-3-oxo-6-syn-(3'α-hydroxyoctyl)-7-anti-acetoxy-cis-bicyclo[3.3.0]octane

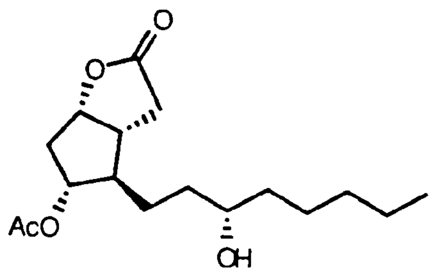

Platinum dioxide (2.0 g) was added to ethanol (300 ml). The mixture was stirred for 10 mins. under atmosphere of argon. A solution of 2-oxa-3-oxo-6-syn-(3'α-hydroxy-1'-trans-octenyl)-7-anti-acetoxy-cis-bicyclo[3.3.0]octane (18.25 g) in ethanol (200 ml) was added dropwise to the solution. The reaction mixture was stirred for 2 hrs. at room temperature under atmosphere of hydrogen. After reaction, catalyst was removed by filtration with CELITE (TM). The filtrate was evaporated to give the title compound (18.3 g) having the following physical data:
Appearance : pale dark brown oil;
TLC : Rf 0.36 (ethyl acetate : hexane = 4 : 1).

Reference Example 2

Synthesis of 2-oxa-3-oxo-6-syn-(3'α-t-butyldimethylsilyloxyoctyl)-7-anti-acetoxy-cis-bicyclo[3.3.0]octane

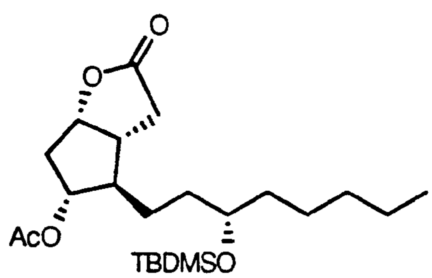

Imidazole (12.0 g) and t-butyldimethylchlorosilane (18.3 g) were added to a solution of the compound (12.0 g) prepared in reference example 1 in dimethylformamide (40 ml). The solution was stirred for 1.5 hrs at room temperature under atmosphere of argon. After reaction, the reaction mixture was diluted with ethyl acetate (100 ml). The diluted solution was washed with 2N hydrochloride, a saturated aqueous solution of sodium hydrogencarbonate and saturated brine, respectively, dried over anhydrous magnesium sulfate and evaporated. The residue was purified by column chromatography on silica gel (ethyl acetate : hexane = 1 : 5) to give the title compound (17.03 g) having the following physical data:
Appearance : colorless oil;
TLC : Rf 0.69 (ethyl acetate : hexane = 1 : 1).

Reference Example 3

Synthesis of 2-oxa-3-oxo-6-syn-(3'α-t-butyldimethylsilyloxyoctyl)-7-anti-hydroxy-cis-bicyclo[3.3.0]octane

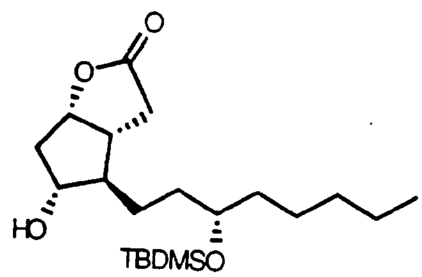

Potassium carbonate (anhydrous, 5.53 g) was added to a solution of the compound (17.03 g) prepared in reference example 2 in methanol (160 ml). The mixture was stirred for 1 hr. at room temperature under atmosphere of argon. Methanol was removed from the mixture. The residue was dissolved in diethyl ether (150 ml) and the solution was filtered. The filtrate was evaporated to give the title compound (15.4 g) having the following physical data:
TLC : Rf 0.41 (ethyl acetate : hexane = 1 : 1).

Reference Example 4

Synthesis of 2-oxa-3-oxo-6-syn-(3'α-t-butyldimethylsilyloxyoctyl)-7-anti-tetrahydropyran-2-yloxy-cis-bicyclo[3.3.0]octane

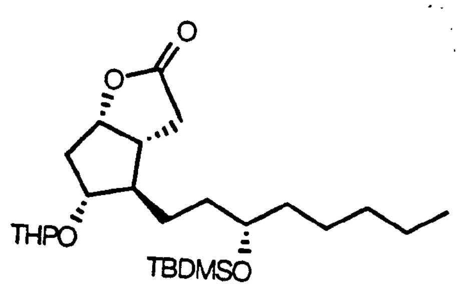

Dihydropyran (15.4 g) and camphorsulfonic acid (464 mg) were added to a solution of the compound (15.4 g) prepared in reference example 3. The solution was stirred for 1.5 hr. at room temperature under atmosphere of argon. The reaction mixture was diluted with methylene chloride (100 ml). The solution was washed with a saturated aqueous solution of sodium hydrogencarbonate, dried over anhydrous magnesium sulfate, and evaporated. The residue was purified by column chromatography on silica gel (ethyl acetate : hexane = 1: 5) to give the title compound (15.06 g) having the following physical data:
Appearance : colorless oil;
TLC : Rf 0.75 (ethyl acetate : hexane = 1 : 1).

Reference Example 5

2-Oxa-3-hydroxy-6-syn-(3'α-t-butyldimethylsilyloxyoctyl)-7-anti-tetrahydropyran-2-yloxycis-bicyclo[3.3.0] octane

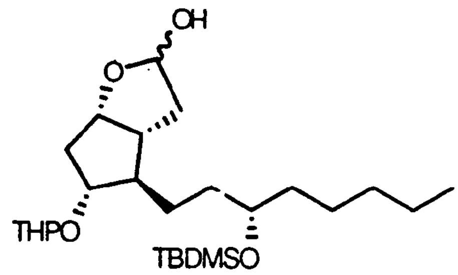

A solution of the compound (15.06 g) prepared in reference example 4 in toluene (150 ml) was cooled at -78°C. A 1.5 M solution of diisobutyl aluminum hydride in toluene (28 ml) was added to the solution dropwise. The reaction mixture was stirred for 15 mins. at -78°C, and methanol (2 ml) was added to the solution. Water was added to the mixture, the mixture was stirred for 1 hr. at room temperature. Precipitates were removed from the mixture by filtration. The filtrate was evaporated to give the title compound (15.0 g) having the following physical data:
Appearance : colorless oil;
TLC : Rf 0.47 (ethyl acetate : hexane = 1 : 1).

Reference Example 6

Synthesis of 9α-hydroxy-11α-tetrahydropyran-2-yloxy-15α-t-butyldimethylsilyloxy-5Z-prostenoic acid

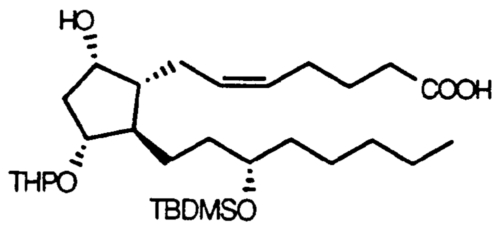

Potassium t-butoxide (20.83 g) was added to a solution of well dried 4-carboxybutyl triphenylphosphine bromide (42.5 g) in toluene (350 ml). The mixture was stirred for 40 mins. at 80°C. The reaction mixture was cooled to 0°C. A solution of the compound (15.0 g) prepared in reference example 5 in toluene (115 ml) was added dropwise to the above solution. The reaction mixture was stirred for 1 hr. at 0°C. The mixture was diluted with ethyl acetate (200 ml). The solution was washed with 1N hydrochloride and saturated brine, respectively, dried over anhydrous magnesium sulfate and evaporated to give the title compound (36.3 g) having the following physical data:
Appearance : pale dark brown oil;
TLC : Rf 0.30 (ethyl acetate : hexane = 1 : 1).

Reference Example 7

Synthesis of 9α-hydroxy-11α-tetrahydropyran-2-yloxy-15α-t-butyldimethylsilyloxy-5Z-prostenoic acid isopropyl ester

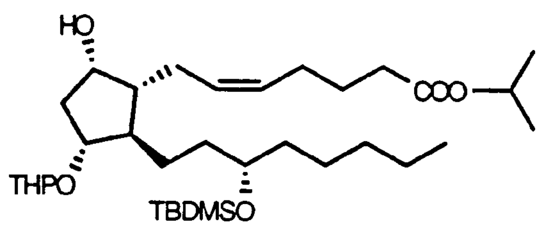

The compound (36.3 g) prepared in reference example 6 and 2-propanol (100 ml) were added to anhydrous

tetrahydrofuran (400 ml). The solution was cooled. Under atmosphere of argon, 2-chloro-1-methyl-pyridinium iodide (33.5 g), diisopropyl ethylamine (46 ml) and 4-dimethylaminopyridine (4.0 g) were added to the solution. The mixture was stirred for 2 hrs. at room temperature. The reaction solution was diluted with ethyl acetate (400 ml). The solution was washed with 1N hydrochloride, a saturated aqueous solution of sodium hydrogencarbonate and a saturated brine, respectively, dried over anhydrous magnesium sulfate and evaporated. The residue was purified by column chromatography on silica gel (ethyl acetate:hexane = 1:5) to give the title compound (14.32 g) having the following physical data:
Appearance : colorless oil;
TLC : Rf 0.20 (ethyl acetate : hexane = 1 : 5).

Reference Example 8

Synthesis of 9β-formyloxy-11α-tetrahydropyran-2-yloxy-15α-t-butyldimethylsilyloxy-5Z-prostenoic acid isopropyl ester

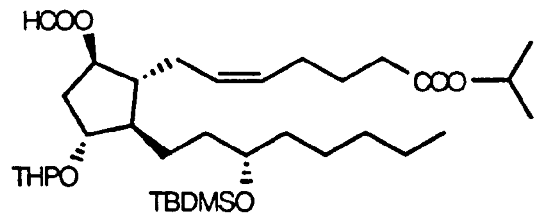

The compound (14.32 g) prepared in reference example 7 and formic acid (1.63 ml) were dissolved in anhydrous tetrahydrofuran (155 ml). Under atmosphere of argon, triphenylphosphine (11.33 g) and diethyl azodicarboxylate (6.8 ml) were added to the solution at 0°C. The reaction mixture was stirred for 50 mins. at 0°C. The reaction solution was diluted with ethyl acetate (200 ml). The solution was washed with a saturated aqueous solution of sodium hydrogencarbonate and a saturated brine, respectively, dried over anhydrous magnesium sulfate and evaporated. The residue was purified by column chromatography on silica gel (ethyl acetate : hexane = 1:10) to give the title compound (9.9 g) having the following physical data:
Appearance : colorless oil;
TLC : Rf 0.38 (ethyl acetate : hexane = 1:5).

Reference Example 9

Synthesis of 9β-hydroxy-11α-tetrahydropyran-2-yloxy-15α-t-butyldimethylsilyloxy-5Z-prostenoic acid isopropyl ester

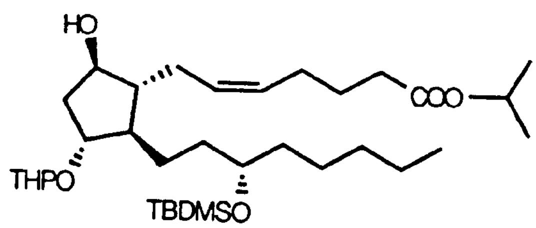

Anhydrous sodium carbonate (3.26 g) was added to a solution of the compound (4.9 g) prepared in reference example 8 in isopropanol (47 ml). The solution was stirred for 4 hrs. at 50°C under atmosphere of argon. The precipitates in the solution were removed by filtration. The filtrate was evaporated to give the title compound (4.7 g) having the following physical data:
TLC : Rf 0.12 (ethyl acetate : hexane = 1:5).

Example 1

Synthesis of 13,14-dihydro-PGF2β isopropyl ester

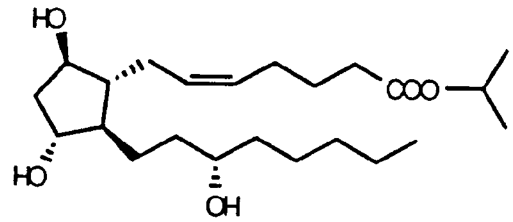

A mixture of 65% acetic acid-tetrahydrofuran (10:1, 80 ml) was added to the compound (4.7 g) prepared in reference example 9. The mixture was stirred for 40 mins. at 80°C. The reaction mixture was cooled to room temperature and diluted with ethyl acetate (150 ml). The solution was washed with a saturated aqueous solution of potassium hydrogencarbonate and a saturated brine, respectively and evaporated. The residue was purified by column chromatography on silica gel (ethyl acetate : hexane = 4:1->9:1) to give the title compound (2.51 g) having the following physical data:
Appearance : white crystal;
TLC : Rf 0.41 (methanol : methylene chloride = 1 : 10);
NMR : δ 5.48 (2H, m), 4.99 (1H, septet, J=6Hz), 4.03 (2H, m), 3.60 (1H, br), 2.33-1.20 (27H, m), 1.22 (6H, d, J=6Hz), 0.86 (3H, t, J=7Hz);
IR : ν 3270, 3012, 2982, 2927, 2857, 1729, 1459, 1377, 1353, 1317, 1250, 1188, 1146, 1112, 1038, 968, 949, 864, 823, 724, 521 cm-1;
mp. : 74.5°C.

Example 2

Synthesis of 13,14-dihydro-prostaglandin F2β

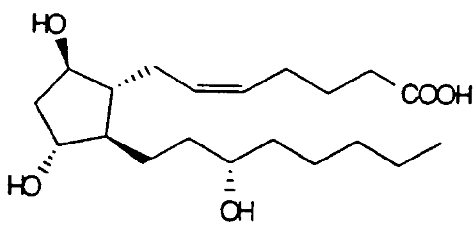

The compound prepared in reference example 8 (400 mg) was dissolved in 65%-acetic acid-THF (20+2 ml). The solution was stirred for 1 hr. at 50°C. After cooling to room temperature, the solution was poured into a saturated aqueous solution of sodium hydrogencarbonate (30 ml) which was ice-cooled. The mixture was extracted with ethyl acetate. The combined oily layers were washed with saturated brine, dried over magnesium sulfate anhydride, and evaporated. The residue was dissolved in methanol (15 ml).

To the solution, a 2N aqueous solution of lithium hydroxide (93 ml) was added. The mixture was stirred for 1.5 hr. at room temperature and for 40 mins. at 50°C. The mixture was evaporated. Water (20 ml) was added to the residue. The solution was extracted with ether. The water layer was adjusted to pH 3 with 2N hydrochloric acid. The mixture was extracted with ethyl acetate. The organic layer combined was dried over anhydrous magnesium sulfate and evaporated. The residue was purified with column chromatography on silica gel (acetic acid/ethyl acetate/hexane) to give the title compound having the following physical data:
Appearance : white crystal;
TLC : Rf 0.19 (acetic acid : methanol : methylene chloride = 1:10:100);
NMR : δ 5.60-5.35 (2H, m), 4.00-3.75 (2H, m), 3.60-3.40 (1H, br), 2.40-2.00 (6H, m), 1.87-1.20 (18H, m), 0.89 (3H,t).

Formulation example

Conc. glycerol and Polysorbate 80 were added to sterilized water. 13,14-dihydro-PGF2β isopropyl ester was dissolved to the solution to obtain following eye drop solutions.

| Formulation 1 | |
|---|---|
| 13,14-dihydro-PGF2β isopropyl ester | 0.001 g |
| Conc. glycerol | 2.5 g |
| Polysorbate 80 | 0.2 g |
| sterilized water to 100 ml | |

| Formulation 2 | |
|---|---|
| 13,14-dihydro-PGF2β isopropyl ester | 0.01 g |
| Conc. glycerol | 2.5 g |
| Polysorbate 80 | 0.2 g |
| sterilized water to 100 ml | |

| Formulation 3 | |
|---|---|
| 13,14-dihydro-PGF2β isopropyl ester | 0.1 g |
| Conc. glycerol | 2.5 g |
| Polysorbate 80 | 0.2 g |
| sterilized water to 100 ml | |

| Formulation 4 | |
|---|---|
| 13,14-dihydro-PGF2β isopropyl ester | 0.2 g |
| Conc. glycerol | 2.5 g |
| Polysorbate 80 | 0.2 g |
| sterilized water to 100 ml | |

## Claims

1. 13,14-dihydro-prostaglandin F2β and its isopropyl ester of formula:

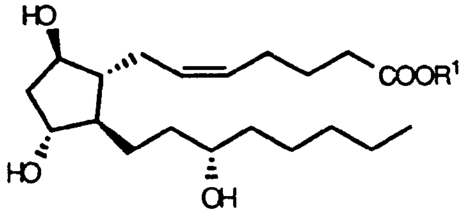

(I)

wherein $R^1$ is hydrogen or isopropyl, or a cyclodextrin clathrate thereof or, when $R^1$ is hydrogen, a pharmaceutically acceptable salt thereof.

2. A compound according to claim 1, which is 13,14-dihydro-PGF2β isopropyl ester.

3. A compound according to claim 1, which is 13,14-dihydro-PGF2β.

4. A process for the preparation of 13,14-dihydro-prostaglandin F2β isopropyl ester of the formula:

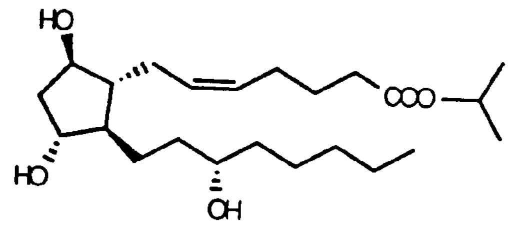

(IB)

which comprises hydrolysing a compound of formula:

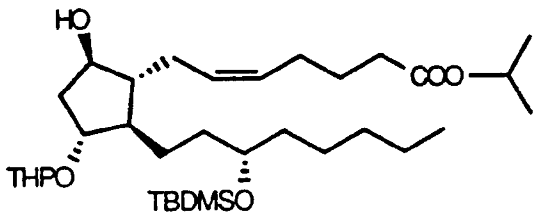

(II)

wherein THP is tetrahydropyran-2-yl and TBDMS is t-butyldimethylsilyl, under acid conditions.

5. A process for the preparation of 13,14-dihydroprostaglandin F2β of the formula:

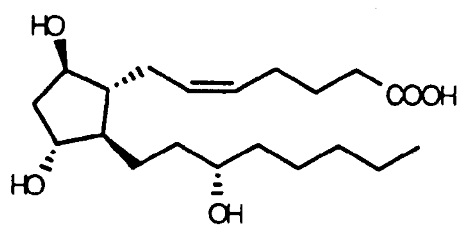

(IA)

which comprises hydrolysing a compound of formula:

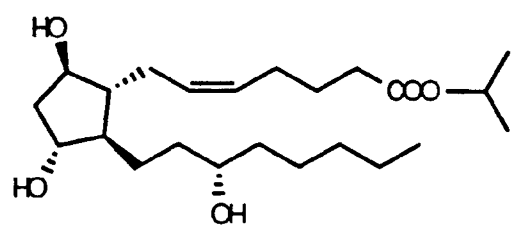

(IB)

under alkaline conditions.

6. A pharmaceutical composition which comprises, as active ingredient, an effective amount of a compound as claimed in claim 1 with a pharmaceutical carrier or coating.

7. A compound as claimed in claim 1, 2 or 3 for use in the prevention and/or treatment of glaucoma.

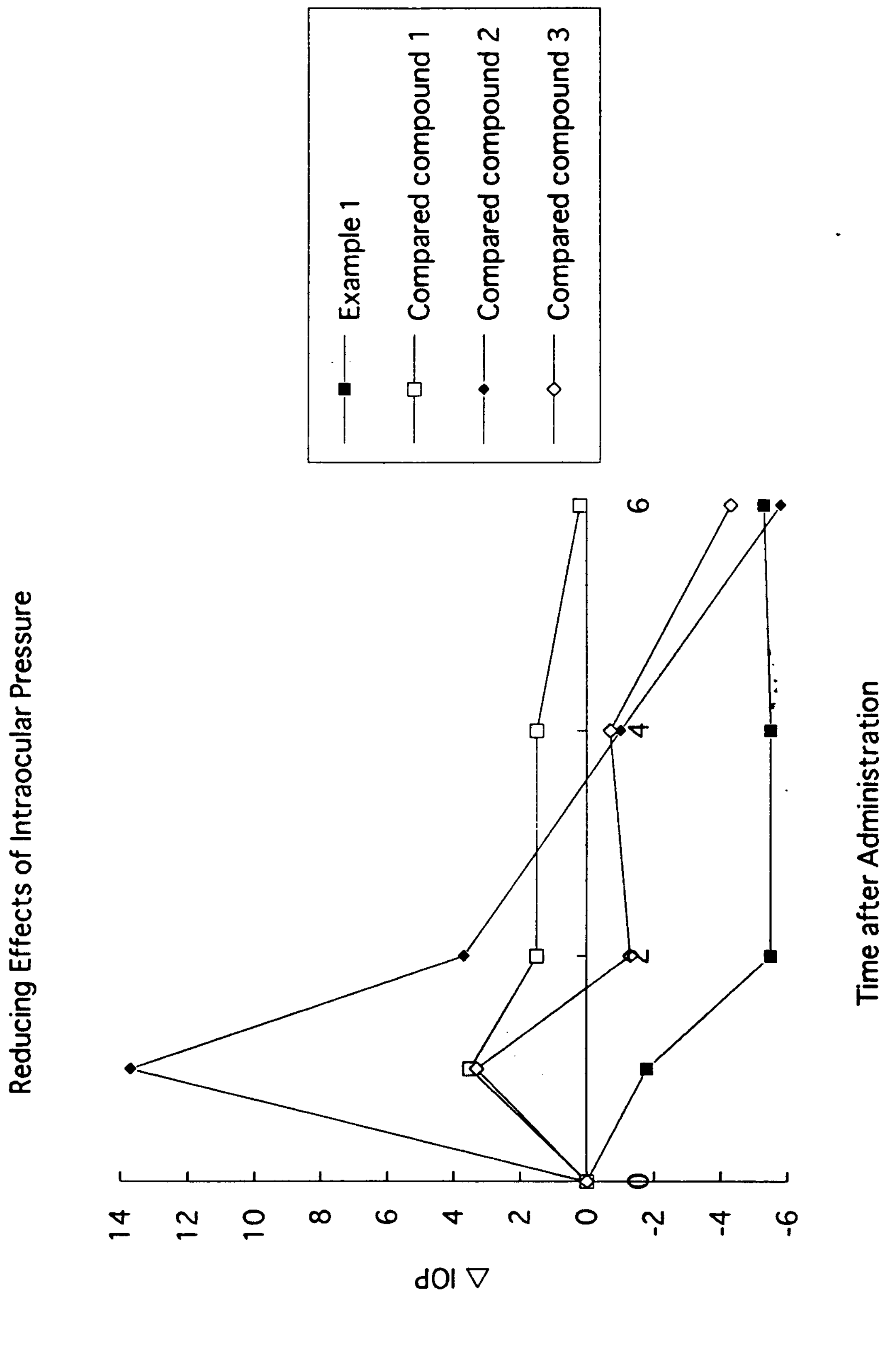
Reducing Effects of Intraocular Pressure
Δ IOP
14
12
10
8
6
4
2
0
-2
-4
-6
0
2
4
6
Time after Administration
Example 1
Compared compound 1
Compared compound 2
Compared compound 3

European Patent Office

# EUROPEAN SEARCH REPORT

Application Number
EP 94 30 4299

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| Y | WO-A-89 03384 (PHARMACIA AB)<br>* page 5 - page 7; claims *<br>--- | 1-7 | C07C405/00<br>//A61K31/557 |
| Y | WO-A-92 10193 (ALLERGAN INC)<br>* page 6, line 9 - line 16; claims 1,12,18 *<br>--- | 1-7 | |
| D,A | EP-A-0 093 380 (THE TRUSTEES OF COLUMBIA UNIVERSITY IN THE CITY OF NEW YORK)<br>* table 3 *<br>--- | 1-7 | |
| X | EP-A-0 015 658 (AMERICAN CYANAMID COMPANY)<br>* page 3, line 33 - page 5, line 32; claim 1 *<br>----- | 1-7 | |
| | | | TECHNICAL FIELDS SEARCHED (Int.Cl.5) |
| | | | C07C<br>A61K |

The present search report has been drawn up for all claims

| Place of search | Date of completion of the search | Examiner |
|---|---|---|
| THE HAGUE | 2 August 1994 | Berte, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)